# EUROPÄISCHE PATENTSCHRIFT

(11) **EP 3 258 981 B1**
(45) Veröffentlichungstag und Bekanntmachung des Hinweises auf die Patenterteilung: **18.03.2020**
(21) Anmeldenummer: 16703548.4
(22) Anmeldetag: 09.02.2016
(51) Int. Cl.: A61M 1/06

(54) **ADAPTER MIT MEDIENTRENNMEMBRAN FÜR EINE BRUSTHAUBE**
ADAPTER WITH MEDIA SEPARATION MEMBRANE FOR A BREASTSHIELD
ADAPTATEUR DOTÉ D'UNE MEMBRANE DE SÉPARATION DE FLUIDES POUR UNE TÉTERELLE

(30) Priorität: 20.02.2015 EP 15155892
(43) Veröffentlichungstag der Anmeldung: 27.12.2017
(73) Patentinhaber: Medela Holding AG, 6340 Baar (CH)
(72) Erfinder: RIGERT, Mario, 6033 Buchrain (CH); EMMENEGGER, Bernhard, 6006 Luzern (CH)
(74) Vertreter: Clerc, Natalia
(86) Internationale Anmeldenummer: PCT/EP2016/052711
(87) Internationale Veröffentlichungsnummer: WO 2016/131677

(56) Entgegenhaltungen:
- EP-A1- 1 818 067
- WO-A1-2014/094187
- CN-U- 202 184 978
- CN-U- 202 942 469
- US-A1- 2010 324 477
- US-A1- 2012 071 820

## Beschreibung

### TECHNISCHES GEBIET

Die vorliegende Erfindung betrifft einen Adapter mit einer Medientrennmembran für eine Brusthaube einer Brustpumpe zum Abpumpen von menschlicher Muttermilch.

### STAND DER TECHNIK

Brustpumpensysteme zum Abpumpen von menschlicher Muttermilch sind wohlbekannt. Sie weisen eine manuell oder elektromotorisch betriebene Vakuumpumpe, mindestens eine Brusthaube zur Auflage auf die Mutterbrust und einen Milchsammelbehälter auf, in welchem die abgepumpte Milch aufgefangen wird. Die Brusthaube ist mit der Vakuumpumpe direkt oder über eine Saugleitung verbunden, so dass in der Brusthaube ein zyklisch variierender Unterdruck an die Brust angelegt werden kann, um die Milch aus der Brust abzupumpen.

Bekannte Brusthauben sind in einem Adapter lösbar gehalten oder fest mit diesem verbunden. Der Adapter dient zur Verbindung mit einem Milchsammelbehälter und mit einer Vakuumpumpe. Die Pumpe kann dabei über eine Saugleitung mit dem Adapter verbunden sein oder direkt an diesem angeordnet sein. Auch der Milchsammelbehälter, üblicherweise ein Beutel oder eine Flasche, ist direkt lösbar am Adapter befestigt oder über eine Milchleitung mit diesem verbunden.

In WO 01/47577 sind derartige Brusthauben gezeigt. Sie sind über je einen Saugschlauch mit der Vakuumpumpe verbunden, wobei am pumpenseitigen Ende des Saugschlauchs eine Kappe mit einer daran gehaltenen Medientrennmembran vorhanden ist. Diese Kappe wird zusammen mit der Medientrennmembran auf die Pumpmembran der Vakuumpumpe aufgestülpt und bildet zusammen mit der Pumpmembran die Pumpkammer. Die Medientrennmembran schmiegt sich an die Oberfläche der Pumpmembran an und bewegt sich gemeinsam mit dieser in zyklischen Bewegungen. Dank der Medientrennmembran wird der Pumpenbereich vor Verunreinigungen und Kontamination geschützt, da sie das Medium "Luft in der Vakuumpumpe" vom Medium "Luft und Milch in der Brusthaube" trennt. Sie schützt insbesondere vor in den Saugschlauch gelangende abgepumpte Muttermilch und Bakterien. Dieselbe Pumpe kann dadurch von mehreren Müttern verwendet werden. Sie lässt sich insbesondere in Spitälern optimal einsetzen und sie kann vermietet werden.

WO 2011/035447 zeigt einen sehr einfachen, zylinderförmigen Adapter, welcher mit lediglich einer Leitung verbunden ist. Diese Leitung dient sowohl als Saug- wie auch als Milchleitung. Die Medientrennmembran, welche auch die Milch in einen Milchsammelbehälter fördert, ist am pumpenseitigen Ende der Saugleitung und vor der Vakuumpumpe angeordnet. Sie überträgt den zyklisch erzeugten Unterdruck über die Saugleitung an die Brusthaube und somit zur Mutterbrust.

Ferner sind Brustpumpensysteme bekannt, bei welchen die Medientrennmembran am brusthaubenseitigen Ende des Saugschlauchs in einem Adapter der Brusthaube angeordnet ist. Auch diese Medientrennmembran überträgt den zyklisch erzeugten Unterdruck von der Vakuumpumpe in die Brusthaube und schützt den Saugschlauch sowie die Vakuumpumpe vor Verunreinigungen.

US 5 941 847 offenbart eine derartige faltbare Medientrennmembran, welche zylinderförmig ausgebildet ist und im zylinderförmigen Stutzen der Brusthaube angeordnet ist.

WO 2014/094187 beschreibt eine Medientrennmembran, welche sich mindestens zeitweise in demjenigen Brusthaubenbereich befindet, in welchen auch die Brustwarze hineinragt.

Die Medientrennmembran gemäss US 2010/0324477 ist in einem oberen Bereich des Adapters angeordnet und mit einem Drehverschlussdeckel überdeckt. Die Membran ist kreisförmig ausgebildet mit ringförmigen Erhebungen und Vertiefungen.

US 2004/0087898 zeigt eine halbkugelförmige Medientrennmembran, welche zwischen zwei Adapterteilen dichtend gehalten ist. Der brusthaubenferne Adapterteil weist einen Behälteranschluss zur Befestigung eines Milchsammelbehälters auf. In diesem Anschlussteil ist ein Rückschlagventil angeordnet. Die Medientrennmembran lässt sich montieren, indem der brusthaubenseitige Adapterteil um eine Achse geschwenkt wird.

In WO 2008/057218 wird vorgeschlagen, anstelle einer faltbaren Medientrennmembran eine vorgespannte Membran zu verwenden. Der Adapter ist relativ aufwändig gestaltet.

CN 202 184 978 U und CN 202 942 469 U zeigen Brustpumpenanordnungen mit einer Kammer, welche eine Verbindungsöffnung zur Brusthaube aufweist. Die Milchauslassöffnung ist jeweils im unteren Bereich der Anordnung bei einem Entenschnabelventil angeordnet.

Weiche, in sich zusammenfallende Medientrennmembranen benötigen eine Rückstellkraft durch die Vakuumpumpe, um ihre ursprüngliche Form zurückzuerhalten. Es besteht stets die Gefahr, dass sie im Verlauf des Pumpvorgangs ihre Originalform nicht mehr vollständig einnehmen und somit den zyklisch angelegten Unterdruck nicht mehr optimal übertragen können.

Steifere Medientrennmembranen haben den Nachteil, dass sie einen grösseren Druckabfall über der Membran aufweisen. Dies ist insbesondere bei grossen Schild- oder Trichtergrössen problematisch, da in diesen Fällen das Pumpenschöpfvolumen des Brustpumpensystems oft nicht mehr gross genug ist, um einen genügenden Unterdruck an die Mutterbrust anzulegen.

### DARSTELLUNG DER ERFINDUNG

Es ist deshalb eine Aufgabe der Erfindung, einen Adapter mit einer Medientrennmembran zu schaffen, welcher einen möglichst kleinen Druckabfall über der Medientrennmembran ermöglicht, wobei sich die Medientrennmembran trotzdem zuverlässig in ihre ursprüngliche Form zurückstellen lässt.

Diese Aufgabe löst ein Adapter gemäss Patentanspruch 1.

Im erfindungsgemässen Adapter für eine Brusthaube einer Brustpumpe zum Abpumpen menschlicher Muttermilch ist die Brusthaube haltbar oder sie ist mit diesem einstückig verbunden. Der Adapter weist einen Sauganschluss zur Verbindung mit einer Vakuumpumpe und einen Milchanschluss zur Verbindung mit einem Milchsammelbehälter auf. Der Adapter weist ferner eine Kammer auf, aus welcher der Sauganschluss und eine Milchauslassöffnung herausführen, wobei die Kammer ferner eine zur Brusthaube führende brusthaubenseitige Öffnung mit einem ersten Durchmesser und eine brusthaubenferne Öffnung mit einem zweiten Durchmesser aufweist. Der Adapter weist zudem eine flexible Medientrennmembran auf, welche in der Kammer aufgenommen ist und die Kammer in einen pumpenseitigen und einen brusthaubenseitigen Bereich trennt zwecks Übertragung eines von der Vakuumpumpe erzeugten Unterdrucks in die Brusthaube und zwecks Schutz vor Kontamination der Vakuumpumpe. Die Kammer verjüngt sich zur brusthaubenseitigen Öffnung hin und weist die Form einer Kugelkalotte auf, welche in ihrem sich verjüngenden Bereich abgeschnitten ist. Die Medientrennmembran weist im Wesentlichen die Form einer abgeflachten Kugelkalotte mit einer ebenen Oberseite auf und die ebene Oberseite der Medientrennmembran ist der brusthaubenseitigen Öffnung der Kammer zugewandt.

Diese spezielle Form der Kammer, in welcher der von der Vakuumpumpe erzeugte zyklische Unterdruck mediengetrennt übertragen wird, und die dazu passende Form der Medientrennmembran ermöglichen die Verwendung einer sehr dünnen, vorzugsweise biegeschlaffen Medientrennmembran. Dadurch ist der Druckabfall über der Membran minimiert. Er beträgt üblicherweise 1 bis maximal 10 mmHg.

Ein weiterer Vorteil ist, dass der Adapter möglichst einfach, mit einem Minimum an Teilen und somit kostengünstig ausgebildet werden kann. Das Zusammensetzen des Adapters ist für die Mutter leichtverständlich und benötigt kaum Zeit. Insbesondere die Medientrennmembran lässt sich auf einfache Art und Weise auswechseln, reinigen und wieder in die Kammer einsetzen. Der gesamte Adapter lässt sich optimal reinigen, da er mehrheitlich aus grossen Flächen ohne Unterbrechungen besteht und vor Allem grosse, gut zugängliche Öffnungen und einfach gestaltete Dichtungskanten und -flächen aufweist.

Die Funktionsweise der Medientrennmembran ist optimiert, wenn die ebene Oberseite der Medientrennmembran einen Durchmesser aufweist, welcher annähernd dem Durchmesser der brusthaubenseitigen Öffnung entspricht.

Vorzugsweise verjüngt sich die Kammer im Bereich der brusthaubenseitigen Öffnung stufenlos bis zur Öffnung hin. Dies optimiert die Funktionsweise und erleichtert die Reinigung.

Vorzugsweise liegt die Medientrennmembran ohne Unterdruckbeaufschlagung im Wesentlichen an einer Innenseite der Kammer unbelastet an. In dieser bevorzugten Ausführungsform ist die Medientrennmembran somit insbesondere nicht vorgespannt und die Kammer bildet somit im Wesentlichen das Gegenstück zur Medientrennmembran.

Vorzugsweise ist die Medientrennmembran biegeschlaff ausgebildet. Sie braucht eine Rückstellkraft, um während des Pumpzyklus genügend schnell ihre ursprüngliche Gestalt zurückzuerhalten, d.h. in diesem Fall ihre kugelkalottenförmige Gestalt. Diese Rückstellkraft wird durch die Vakuumpumpe gewährleistet, indem das Vakuum abgebaut wird.

Erfindungsgemäß weist die Medientrennmembran eine Vertiefung auf, welche einen Teil eines Milchkanals zwischen der brusthaubenseitigen Öffnung und dem Milchanschluss bildet. Dank dieser Vertiefung kann sich die Medientrennmembran an der Innenseite der Kammer anliegend bis annähernd oder bis ganz zur brusthaubenseitigen Öffnung hin erstrecken und trotzdem kann die Milch ungehindert über die Kammer zum Milchanschluss hin fliessen. Das Totvolumen im Adapter ist dadurch minimiert.

Vorzugsweise befindet sich diese Vertiefung in einem Übergangsbereich zwischen der ebenen Oberseite und einer gekrümmten Mantelfläche der abgeflachten Kugelkalotte der Medientrennmembran.

In einer bevorzugten Ausführungsform weist die Kammer in ihrem kugelkalottenförmigen Bereich eine Milchauslassöffnung auf. Vorzugsweise weist die Medientrennmembran eine flexible Ventilklappe auf, welche diese Milchauslassöffnung verschliesst. Die einstückige Ausbildung der Medientrennmembran mit der Ventilklappe vereinfacht den Aufbau des Adapters zusätzlich. Es ist kein separates Teil für das Milchventil notwendig. Es lässt sich gemeinsam mit der restlichen Medientrennmembran montieren, was die Montage erleichtert. Zudem ist kein kleines separates Ventilteil vorhanden, welches bei der Montage durch die Mutter vergessen werden könnte, welches beim Reinigen irrtümlich den Abfluss des Spülbeckens hinuntergespült werden könnte oder welches von einem Kleinkind verschluckt werden könnte. Des Weiteren gibt die Ventilklappe die genaue Position vor, in welcher die Medientrennmembran in die Kammer eingelegt werden soll. Auch dies erleichtert das Zusammensetzen.

In einer bevorzugten Ausführungsform weist die Medientrennmembran einen kreisförmigen Umfangsrand auf, welcher dicker ausgebildet ist als die abgeflachte Kugelkalotte und welcher mindestens abschnittsweise mit einem Positionierring versehen ist. Die Dichtung ist einfach aufgebaut, aber äusserst wirkungsvoll. Sie lässt sich durch einen einfachen Reibschluss erzielen. Zudem gibt der verdickte Rand der Medientrennmembran Stabilität, so dass sie bei der Montage gut in der Hand liegt.

Vorzugsweise weist der Adapter einen brusthaubenseitigen Grundkörper und einen brusthaubenfernen Deckel auf, wobei die Kammer im Grundkörper angeordnet ist und wobei der Deckel die brusthaubenferne Öffnung der Kammer verschliesst. Dadurch ist die Kammer einfach zugänglich und die Medientrennmembran kann auf einfache Art und Weise eingesetzt und wieder entfernt werden.

In einer bevorzugten Ausführungsform weist der Grundkörper einen kreisförmig umlaufenden ersten Dichtungsrand auf, über welchen die Medientrennmembran stülpbar ist, wobei der mindestens teilweise umlaufende Positionierring diesen ersten Dichtungsrand umschliesst. Vorzugsweise ist der Positionierring nicht vollständig umlaufend ausgebildet, wobei ein Bereich für ein Schwenkscharnier und/oder für einen Schnappverschluss des Adapters freigestellt ist.

Alternativ oder zusätzlich weist der Deckel einen kreisförmig umlaufenden zweiten Dichtungsrand auf, über welchen die Medientrennmembran mit ihrem Umfangsrand stülpbar ist, wobei der Umfangsrand dichtend am zweiten Dichtungsrand anliegt.

Vorzugsweise ist die Medientrennmembran wahlweise auf dem Deckel oder auf dem Grundkörper befestigbar, wobei die Dichtwirkung beim Schliessen des Deckels beidseitig der Medientrennmembran durch Reibschluss erzielt wird. Der Adapter ist dadurch zuverlässig dicht.

Vorzugsweise bildet der Positionierring der Medientrennmembran bei geschlossenem Deckel eine äussere Oberfläche des Adapters zwischen Deckel und Grundkörper. Der Ring ist somit bei geschlossenem Deckel von aussen sichtbar. Zudem ist der Adapter ohne Medientrennmembran nicht dicht, um Milch abzupumpen. Dadurch wird zweifach verhindert, dass der Adapter ohne Medientrennmembran verwendet und die Vakuumpumpe der Gefahr einer Verunreinigung oder Kontamination ausgesetzt wird. Das Zusammensetzen des Adapters ist erleichtert, wenn der Deckel um eine einzige Schwenkachse schwenkbar am Grundkörper angeordnet ist.

Die Medientrennmembran des oben beschriebenen Adapters weist im Wesentlichen die Form einer abgeflachten Kugelkalotte mit einer ebenen Oberseite und einer Vertiefung auf und verfügt in bevorzugten Ausführungsformen über die oben und nachfolgend beschriebenen Merkmale.

### KURZE BESCHREIBUNG DER ZEICHNUNGEN

Eine bevorzugte Ausführungsform der Erfindung wird im Folgenden anhand der Zeichnungen beschrieben, die lediglich zur Erläuterung dienen und nicht einschränkend auszulegen sind. In den Zeichnungen zeigen:
- Figur 1: eine perspektivische Darstellung eines Brustpumpensystems ohne Vakuumpumpe mit dem erfindungsgemässen Adapter;
- Figur 2: eine Explosionsdarstellung des Brustpumpensystems gemäss Figur 1;
- Figur 3: eine Seitenansicht des Adapters gemäss Figur 1 mit montierter Brusthaube;
- Figur 4: einen Längsschnitt durch den Adapter gemäss Figur 1 mit montierter Brusthaube;
- Figur 5: eine vergrösserte Darstellung des Bereichs A gemäss Figur 4;
- Figur 6: eine perspektivische Darstellung des Adapters gemäss Figur 1 aus brusthaubenseitiger Sicht;
- Figur 7: eine perspektivische Darstellung des Adapters gemäss Figur 1 aus brusthaubenferner Sicht;
- Figur 8: eine brusthaubenferne Ansicht einer erfindungsgemässen Medientrennmembran des Adapters gemäss Figur 1;
- Figur 9: eine brusthaubenseitige Ansicht der Medientrennmembran gemäss Figur 8;
- Figur 10: eine Seitenansicht der Medientrennmembran gemäss Figur 8;
- Figur 11: einen Längsschnitt durch die Medientrennmembran gemäss Figur 8;
- Figur 12: eine perspektivische Ansicht des geöffneten Adapters und
- Figur 13: eine perspektivische Darstellung eines Brustpumpensystems einer Vakuumpumpe und mit dem erfindungsgemässen Adapter.

### BESCHREIBUNG BEVORZUGTER AUSFÜHRUNGSFORMEN

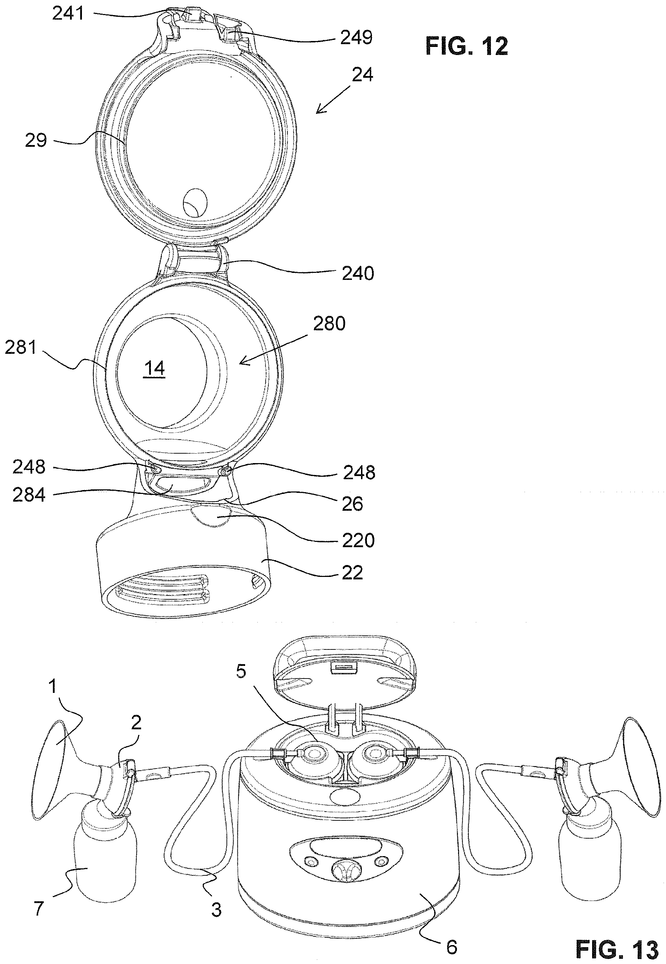

Figur 13 zeigt ein Brustpumpensystem mit einer Vakuumpumpe 6, einer auf eine Pumpmembran der Vakuumpumpe 6 aufgesetzte Schale 5, eine Saugleitung 3, welche die Schale 5 mit einem Adapter 2 verbindet, eine Brusthaube 1, welche mit dem Adapter 2 verbunden ist sowie einen Milchsammelbehälter 7, welcher ebenfalls mit dem Adapter 2 verbunden ist. Vorzugsweise ist die Brusthaube 1 in den Adapter 2 eingesteckt und lösbar mit ihm verbunden. Sie kann jedoch auch einstückig am Adapter 2 angeformt sein. In diesem Beispiel ist eine Doppelpumpe mit zwei Schalen, zwei Saugleitungen und zwei Brusthaubeneinheiten dargestellt.

Schale 5, Adapter 2 und Brusthaube 1 bestehen vorzugsweise aus Kunststoff, wobei die Schale 5 und der Adapter 2 vorzugsweise steif sind und die Brusthaube 1 steif, weich oder halbweich ausgebildet sein kann.

Die hier dargestellte Brusthaube 1 stellt eine eigenständige Erfindung dar. Sie ist in der am gleichen Tag eingereichten Patentanmeldung derselben Anmelderin mit dem Titel "Brusthaube" detailliert beschrieben. Der Inhalt dieser Patentanmeldung wird hiermit durch Referenz in diesen Text aufgenommen.

Die hier dargestellte Schale 5 stellt ebenfalls eine eigenständige Erfindung dar. Sie ist in der am gleichen Tag eingereichten Patentanmeldung derselben Anmelderin mit dem Titel "Schale für eine Brustpumpe" detailliert beschrieben. Der Inhalt dieser Patentanmeldung wird hiermit durch Referenz in diesen Text aufgenommen.

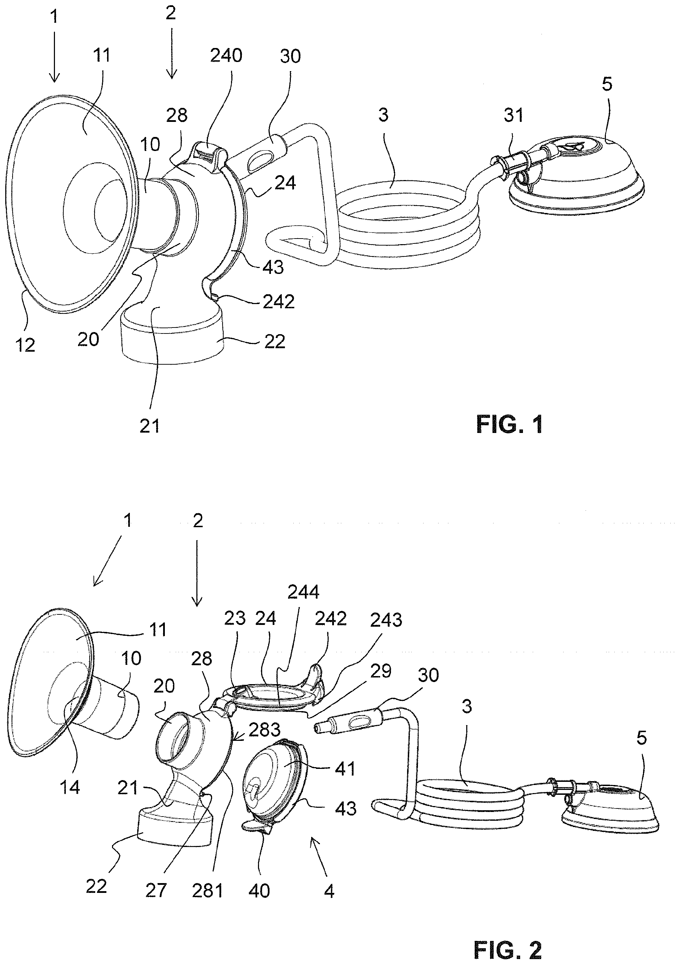

In den Figuren 1 und 2 ist das Brustpumpensystem ohne Vakuumpumpe dargestellt. Die Saugleitung 3 ist vorzugsweise ein flexibler Saugschlauch, welcher über einen nicht zerstörungsfrei pumpenseitigen Verbindungsstecker 31 mit der Schale 5 fest verbunden ist. Am anderen Ende weist die Saugleitung 3 einen adapterseitigen Stecker 30 auf, welcher vorzugsweise lösbar mit einem Sauganschluss 23 des Adapters 2 verbindbar ist. Hier ist sie einsteckbar.

Die Brusthaube 1 weist ein rohrförmiges Anschlussteil 10 und einen daran einstückig angeformten Trichter 11 zur Aufnahme einer menschlichen Mutterbrust und einen Auflagerand 12 zur Auflage auf die menschliche Mutterbrust auf. Auf der äusseren Oberseite des Trichters 11 sind haptische Elemente 13, hier in Form von ringförmigen Erhebungen, vorhanden. Anstelle dieser Brusthaube 1 kann auch eine andere Brusthaube bekannter Art verwendet werden.

Wie in den Figuren 2 bis 4 sowie 6 und 7 gut erkennbar ist, weist der Adapter 2 einen Grundkörper 28 auf, welcher hohl ausgebildet ist und eine Kammer 280 bildet. Am Grundkörper 28 ist ein rohrförmiges Aufnahmeteil 20 angeformt, in welches sich der Anschlussteil 10 der Brusthaube 1 einstecken lässt. Der Grundkörper 28 geht über einen Hals 21 in einen rohrförmigen Milchanschluss 22 über. Dieser ist vorzugsweise mit einem Innengewinde 25 versehen. Es dient zur Verbindung mit einem Milchsammelbehälter, insbesondere einer Milchflasche.

Brusthaube 1 und Adapter 2 bilden einen Durchgang oder Milchkanal 14, welcher sich vom brustnahen Ende der Brusthaube 1 bis zum Milchanschluss 22 erstreckt und somit einen Milchfluss von der Mutterbrust in den Milchsammelbehälter ermöglicht.

Der Adapter 2 weist eine flexible Medientrennmembran 4 auf, welche in der Kammer 280 des Grundkörpers 28 angeordnet ist. Der Adapter 2 weist hierfür einen Deckel 24 auf, damit die Medientrennmembran 4 in das Gehäuse bzw. in den Grundkörper 28 des Adapters 2 eingelegt und zwecks Ersatz oder Reinigung auch wieder daraus entfernt werden kann. Der Deckel 24 ist in diesem Beispiel über ein einziges Schwenkscharnier 240 mit dem Grundkörper 28 verbunden. Das Schwenkscharnier 240 ist vorzugsweise oben angeordnet. Am gegenüberliegenden unteren Ende ist ein Griff 242 angeformt, auf dessen Rückseite eine Rückhaltenase 241 ausgebildet ist. Diese Rückhaltenase 241 greift in eine Rückhaltekante 26 einer Öffnung im Hals 21 des Adapters 2 ein. Dadurch ist ein Schnappverschluss gebildet. Am Milchanschluss 22 ist ferner eine Delle 220 vorhanden, welche das Ergreifen des Griffs 242 erleichtert.

Auf beiden Seiten des Griffs 242 ist je eine nach unten gerichtete, gebogen ausgebildete Flanke 243 als formschlüssiger Abschluss angeformt. Die Flanken 243 und die Rückhaltekante 26 sind vorzugsweise schräg verlaufend ausgebildet.

Der Deckel 24 ist vorzugsweise annähernd flach und im Wesentlichen rund ausgebildet. Der Sauganschluss 23 ist im oberen Bereich des Deckels 24 angeformt.

Die Kammer 280 ist kugelkalottenförmig ausgebildet, wobei ihr verjüngendes Ende abgeschnitten ist und eine erste brusthaubenseitige runde Öffnung 282 bildet. Der Durchmesser dieser brusthaubenseitigen Öffnung 282 entspricht dem Durchmesser des rohrförmigen Aufnahmeteils 20 und ist somit relativ gross.

Das brusthaubenferne Ende der kugelkalottenförmigen Kammer 280 bildet eine zweite brusthaubenferne runde Öffnung 283. Diese brusthaubenferne Öffnung 283 wird vom Deckel 24 überdeckt und verschlossen. Die Kammer 280 kann sich kugelkalottenförmig bis zu dieser brusthaubenfernen Öffnung 283 erstrecken oder sie kann, wie in diesem Beispiel, bereits vorher in eine annähernd kreiszylindrische Form übergehen.

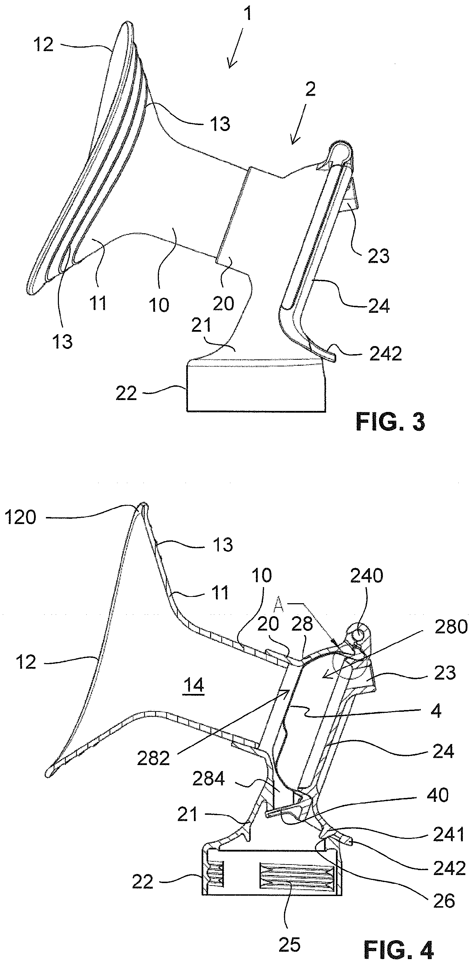

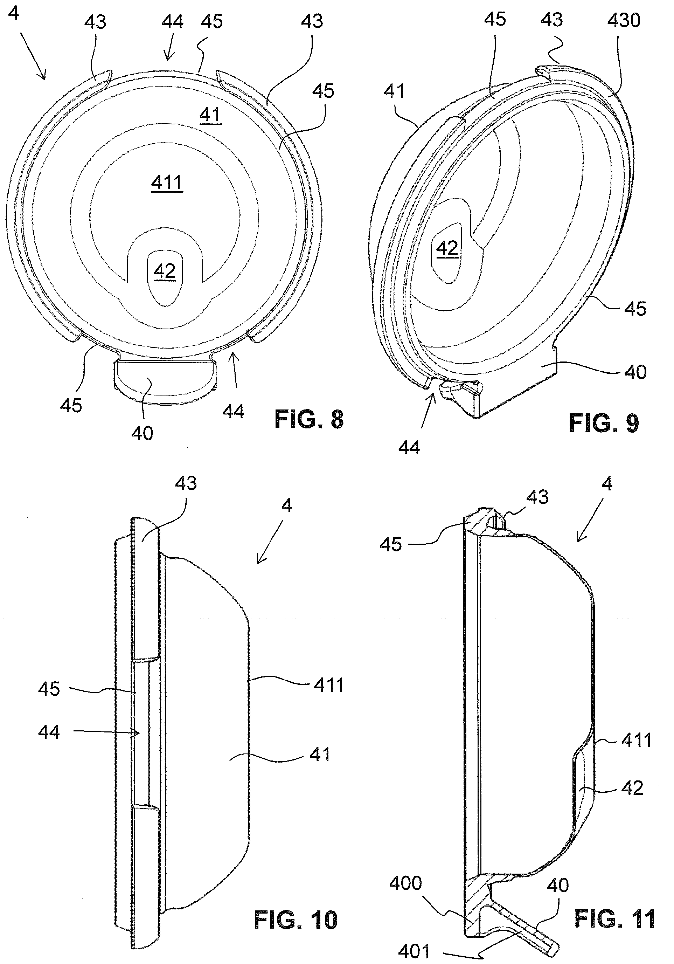

Wie in Figur 4 dargestellt, ist im unteren Bereich der Kammer 280 eine Milchauslassöffnung 284 vorhanden, durch welche abgepumpte Milch in den Milchsammelbehälter fliessen kann. Diese Milchauslassöffnung 284 weist einen zum Milchanschluss 22 hingerichteten umlaufenden Rand auf, welcher einen Ventilsitz bildet. An der Medientrennmembran 4 ist eine Ventilklappe 40 einstückig angeformt, welche gemeinsam mit dem Ventilsitz ein Rückschlagventil bildet und somit das Totvolumen im Adapter 2 begrenzt. Die Ventilklappe 40 steht in einem Winkel, vorzugsweise von ungefähr 55° von einem kreisförmigen Umfangsrand 45 der restlichen Membran 4 ab, wie dies in Figur 11 gut sichtbar ist. Die Ventilklappe 40 weist, wie dies ebenfalls in Figur 11 erkennbar ist, einen Steg 400 auf, welcher an zwei Nasen 249 des Deckels 24 ansteht. Diese Nasen sind in Figur 12 erkennbar. Ein die Ventilklappe 40 umlaufender, nach unten gerichteter Kragen 401 gibt ihr Stabilität.

Die Medientrennmembran 4 weist einen Grundkörper 41 auf, welcher ebenfalls in Form einer abgeflachten Kugelkalotte ausgebildet ist und an welchen die Ventilklappe 40 angeformt ist. Dieser Grundkörper weist vorzugsweise keinerlei Unebenheiten auf, bis auf eine weiter unten erwähnte Vertiefung 42.

Die abgeflachte, rund und ebene Oberfläche des Grundkörpers 41 ist mit dem Bezugszeichen 411 versehen und beispielsweise in den Figuren 8 bis 11 gut erkennbar.

Dieser Grundkörper 41 bildet das Gegenstück zur Kammer 280. Im montierten Zustand liegt der Grundkörper 41 annähernd oder vollständig an der Innenseite der Kammer 280 an, wie dies in Figur 4 erkennbar ist. Die ebene Oberfläche 411 liegt dabei im Bereich der brusthaubenseitigen Öffnung 282 bzw. in dieser Öffnung. Diese Oberfläche 411 weist vorzugsweise annähernd oder genau denselben Durchmesser auf wie diese brusthaubenseitige Öffnung 282.

Im Übergangsbereich zwischen ebener Oberfläche 411 und kugelkalottenförmigen Bereich des Grundkörpers 41 ist die erwähnte Vertiefung 42 vorhanden, welche sich brusthaubenfern als eine Erhöhung darstellt. Diese Vertiefung 42 ist bei korrekt montierter Medientrennmembran 4 im unteren Bereich angeordnet und bildet einen Teil des Milchkanals 14 zwischen Brusthaube 1 und Milchanschluss 22. Eine entsprechende gegenüberliegende Vertiefung im Grundkörper 28 bzw. im Hals 21 kann, muss aber nicht vorhanden sein. In diesem Beispiel ist keine vorhanden.

Die Medientrennmembran 4 ist vorzugsweise aus Silikon hergestellt. Der Grundkörper 41 ist relativ dünn, vorzugsweise biegeschlaff ausgebildet. Typische Dicken betragen 0.2 bis 1.0 mm. Die Ventilklappe 40 ist vorzugsweise dicker ausgebildet.

Der Grundkörper 41 ist als Gegenstück zur Kammer 280 ausgebildet. Weist die Kammer 280 brusthaubenfern einen zylinderförmigen Abschnitt auf, so gilt dies vorzugsweise auch für den Grundkörper 41. Dies ist hier der Fall, wie in den Figuren 10 und 11 erkennbar ist.

Der Grundkörper 41 weist brusthaubenfern den umlaufenden, in sich geschlossenen und kreisförmigen Umfangsrand 45 auf, welcher verdickt ausgebildet ist und dem Grundkörper 41 seine Position und Stabilität gibt.

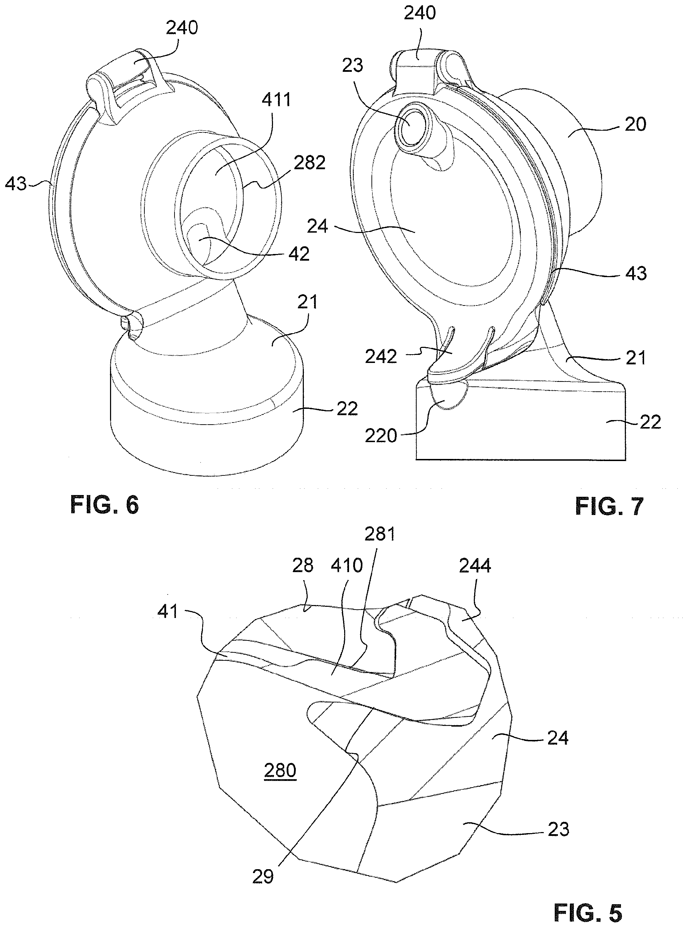

Eine Verdickung ist in Figur 5 mit der Bezugsziffer 410 versehen. Diese Verdickung 410 dichtet gegenüber einem ersten Dichtungsrand 281 des Grundkörpers und gegenüber einem zweiten Dichtungsrand 29 des Deckels. Die Dichtungsränder 281, 29 sind kreisförmig umlaufend und in sich geschlossen ausgebildet und sie verlaufen vorzugsweise konisch zueinander.

Am Umfangsrand 45 ist ferner ein ebenfalls zur Brusthaube hin gerichteter Positionierring 43 angeformt Dieser Positionierring 43 ist nicht vollständig umlaufend ausgebildet, sondern es sind im Bereich des Scharniers 240 und des Griffs 242 Ausnehmungen 44 vorhanden, wie dies in den Figuren 8 bis 10 erkennbar ist.

In Figur 5 ist erkennbar, wie die Dichtung zwischen Deckel 24, Grundkörper 28 des Adapters 2 und der Medientrennmembran 4 im Bereich des Umfangrandes 45 erfolgt. Der Grundkörper 28 weist den bereits erwähnten ersten Dichtungsrand 281 auf, dessen äusserer Durchmesser kleiner ist als der äusserste Durchmesser des Grundkörpers 28 des Adapters. Der erste Dichtungsrand 281 ist durch den Rand der brusthaubenfernen Öffnung 283 gebildet.

Wie in Figur 1 erkennbar ist, ist der Positionierring 43 bei geschlossenem Deckel 24 zwischen den zwei Teilen des Adapters 2 eingeklemmt und bildet einen Teil der äusseren Oberfläche des Adapters 2. Die Medientrennungsmembran 4 ist somit von aussen sichtbar. Zudem ist aufgrund der Ausbildung der Dichtungsflächen, -ränder und -lippe der Adapter 4 nicht dicht, wenn die Medientrennungsmembran 4 nicht montiert ist. Des Weiteren lässt sich der Klappdeckel 24 nicht schliessen, wenn die Medientrennmembran 4 nicht in korrekter Drehposition und Lage angeordnet ist. Die Medientrennmembran 4 kann jedoch sowohl auf dem Deckel 24 montiert sein oder auf dem Grundkörper 28. Wird sie auf dem Deckel 24 montiert, so wird sie über den zweiten Dichtungsrand 29 gestülpt, wobei die Ventilklappe 40 zwischen die zwei Nasen des Deckels gelegt bzw. geklemmt wird. Wird sie auf dem Grundkörper 28 montiert, so wird sie über den ersten Dichtungsrand 281 gestülpt, wobei die Ventilklappe 40 zwischen den zwei Stiften 27 gelegt bzw. geklemmt wird.

Der erfindungsgemässe Adapter ermöglicht einen minimalen Druckabfall über der Medientrennungsmembran und gewährleistet eine zuverlässige Rückstellung in ihre ursprüngliche Form.

**BEZUGSZEICHENLISTE**

| | | | |
|---|---|---|---|
| 1 | Brusthaube | 284 | Milchauslassöffnung |
| 10 | Anschlussteil | 29 | zweiter Dichtungsrand |
| 11 | Trichter | | |
| 12 | Auflagekante | 3 | Saugleitung |
| 13 | haptisches Element | 30 | adapterseitiger Verbindungsstecker |
| 14 | Milchkanal | 31 | pumpenseitiger Verbindungsstecker |
| | | | |
| 2 | Adapter | 4 | Medientrennmembran |
| 20 | Aufnahmeteil | 40 | Ventilklappe |
| 21 | Hals | 400 | Steg |
| 22 | Milchanschluss | 401 | Kragen |
| 220 | Delle | 41 | Grundkörper |
| 23 | Sauganschluss | 410 | Verdickung |
| 24 | Deckel | 411 | ebene Oberseite |
| 240 | Schwenkscharnier | 42 | Vertiefung |
| 241 | Rückhaltenase | 43 | Positionierring |
| 242 | Griff | 44 | Ausnehmung |
| 243 | Flanke | 45 | Umfangsrand |
| 244 | Umfangsrand | | |
| 25 | Innengewinde | 5 | Schale |
| 26 | Rückhaltekante | | |
| 27 | Stift | 6 | Vakuumpumpe |
| 28 | Grundkörper | | |
| 280 | Kammer | 7 | Milchsammelbehälter |
| 281 | erster Dichtungsrand | | |
| 282 | brusthaubenseitige Öffnung | | |
| 283 | brusthaubenferne Öffnung | | |

## Patentansprüche

1. Adapter (2) für eine Brusthaube (1) einer Brustpumpe zum Abpumpen menschlicher Muttermilch, wobei die Brusthaube (1) im Adapter (2) haltbar ist oder mit diesem einstückig verbunden ist,
wobei der Adapter (2) einen Sauganschluss (23) zur Verbindung mit einer Vakuumpumpe (6) und einen Milchanschluss (22) zur Verbindung mit einem Milchsammelbehälter (7) aufweist,
wobei der Adapter (2) eine Kammer (280) aufweist, aus welcher der Sauganschluss (23) und eine Milchauslassöffnung (284) herausführen,
wobei die Kammer (280) ferner eine zur Brusthaube (1) führende brusthaubenseitige Öffnung (282) mit einem ersten Durchmesser und eine brusthaubenferne Öffnung (283) mit einem zweiten Durchmesser aufweist,
und wobei der Adapter (2) ferner eine flexible Medientrennmembran (4) aufweist, welche in der Kammer (280) aufgenommen ist und die Kammer (280) in einen pumpenseitigen und einen brusthaubenseitigen Bereich trennt zwecks Übertragung eines von der Vakuumpumpe (6) erzeugten Unterdrucks in die Brusthaube (1) und zwecks Schutz vor Kontamination der Vakuumpumpe, wobei sich die Kammer (280) zur brusthaubenseitigen Öffnung (282) hin verjüngt, wobei die Kammer (280) die Form einer Kugelkalotte aufweist, welche in ihrem sich verjüngenden Bereich abgeschnitten ist, wobei die Medientrennmembran (4) im Wesentlichen die Form einer abgeflachten Kugelkalotte mit einer ebenen Oberseite (411) aufweist und wobei die ebene Oberseite (411) der Medientrennmembran (4) der brusthaubenseitigen Öffnung (282) der Kammer (280) zugewandt ist, **dadurch gekennzeichnet, dass** die Medientrennmembran (4) eine Vertiefung (42) aufweist, welche einen Teil eines Milchkanals (14) zwischen der brusthaubenseitigen Öffnung (282) und dem Milchanschluss (22) bildet.

2. Adapter nach Anspruch 1, wobei die ebene Oberseite (411) der Medientrennmembran (4) einen Durchmesser aufweist, welcher annähernd dem Durchmesser der brusthaubenseitigen Öffnung (282) entspricht.

3. Adapter nach einem der Ansprüche 1 oder 2, wobei sich die Kammer (280) im Bereich der brusthaubenseitigen Öffnung (282) stufenlos bis zur Öffnung hin verjüngt.

4. Adapter nach einem der Ansprüche 1 bis 3, wobei die Medientrennmembran (4) ohne Unterdruckbeaufschlagung im Wesentlichen an einer Innenseite der Kammer (280) unbelastet anliegt.

5. Adapter nach einem der Ansprüche 1 bis 4, wobei die Medientrennmembran (4) biegeschlaff ausgebildet ist.

6. Adapter nach einem der Ansprüche 1 bis 5, wobei sich die Vertiefung (42) in einem Übergangsbereich zwischen der ebenen Oberseite (411) und einer gekrümmten Mantelfläche der abgeflachten Kugelkalotte der Medientrennmembran (4) befindet.

7. Adapter nach einem der Ansprüche 1 bis 6, wobei die Milchauslassöffnung (284) im kugelkalottenförmigen Bereich der Kammer (280) angeordnet ist.

8. Adapter nach Anspruch 7, wobei die Medientrennmembran (4) eine flexible Ventilklappe (40) aufweist, welche diese Milchauslassöffnung (284) verschliesst.

9. Adapter nach einem der Ansprüche 1 bis 8, wobei die Medientrennmembran (4) einen kreisförmigen Umfangsrand (45) aufweist, welcher dicker ausgebildet ist als die abgeflachte Kugelkalotte der Medientrennmembran (4) und welcher mindestens abschnittsweise mit einem Positionierring (43) versehen ist.

10. Adapter nach einem der Ansprüche 1 bis 9, wobei der Adapter (2) einen brusthaubenseitigen Grundkörper (28) und einen brusthaubenfernen Deckel (24) aufweist, wobei die Kammer (280) im Grundkörper (28) angeordnet ist und wobei der Deckel (24) die brusthaubenferne Öffnung (283) der Kammer (280) verschliesst.

11. Adapter nach den Ansprüchen 9 und 10, wobei der Grundkörper (28) einen kreisförmig umlaufenden ersten Dichtungsrand (281) aufweist, über welchen die Medientrennmembran (4) stülpbar ist, wobei der mindestens teilweise umlaufende Positionierring (43) diesen ersten Dichtungsrand (281) umschliesst.

12. Adapter nach einem der Ansprüche 9 bis 11, wobei der Deckel (24) einen kreisförmig umlaufenden zweiten Dichtungsrand (29) aufweist, über welchen die Medientrennmembran (4) mit ihrem Umfangsrand (45) stülpbar ist, wobei der Umfangsrand (45) dichtend am zweiten Dichtungsrand (29) anliegt.

13. Adapter nach den Ansprüchen 11 und 12, wobei der Positionierring (43) der Medientrennmembran (4) bei geschlossenem Deckel (24) eine äussere Oberfläche des Adapters (2) zwischen Deckel (24) und Grundkörper (28) bildet.

14. Adapter nach einem der Ansprüche 10 bis 13, wobei der Deckel (24) um eine einzige Schwenkachse (240) schwenkbar am Grundkörper (28) angeordnet ist.

## Claims

1. Adapter (2) for a breast shield (1) of a breast pump for expressing human breast milk, wherein the breast shield (1) is holdable in the adapter (2) or is integrally connected in one piece to said adapter,
wherein the adapter (2) comprises a suction connection (23) for connection to a vacuum pump (6) and a milk connection (22) for connection to a milk collecting container (7),
wherein the adapter (2) comprises a chamber (280) from which the suction connection (23) and a milk outlet opening (284) lead,
wherein the chamber (280) additionally comprises an opening (282) on the breast shield side which leads to the breast shield (1) having a first diameter and an opening (283) which is remote from the breast shield having a second diameter,
and wherein the adapter (2) additionally comprises a flexible media separating diaphragm (4) which is received in the chamber (280) and separates the chamber (280) into a pump-side region and a breast-shield-side region for the purposes of transmitting a negative pressure generated by the vacuum pump (6) into the breast shield (1) and for the purposes of protecting against contamination of the vacuum pump,
wherein the chamber tapers toward the opening (282) on the breast shield side,
wherein the chamber (280) comprises the form of a spherical calotte which has been cut off in its tapering region,
wherein the media separating diaphragm (4) comprises substantially the form of a flattened spherical calotte with an even top surface (411) and
wherein the even top surface (411) of the media separating diaphragm (4) faces the opening (282) of the chamber (280) on the breast shield side, **characterized in that** the media separating diaphragm (4) comprises an indentation (42) which forms part of a milk channel (14) between the opening (282) on the breast shield side and the milk connection (22).

2. Adapter according to Claim 1, wherein the even top surface (411) of the media separating diaphragm (4) comprises a diameter which corresponds approximately to the diameter of the opening (282) on the breast shield side.

3. Adapter according to either of Claims 1 or 2, wherein the chamber (280) tapers steplessly toward the opening in the region of the opening (282) on the breast shield side.

4. Adapter according to one of Claims 1 to 3, wherein the media separating diaphragm (4) abuts substantially against an inside surface of the chamber (280) in the non-loaded state without negative pressurization.

5. Adapter according to one of Claims 1 to 4, wherein the media separating diaphragm (4) is limp.

6. Adapter according to one of Claims 1 to 5, wherein the indentation (42) is situated in a transition region between the even top surface (411) and a curved lateral surface of the flattened spherical calotte of the media separating diaphragm (4).

7. Adapter according to one of Claims 1 to 6, wherein the milk outlet opening (284) is arranged in the spherical segment-shaped region of the chamber (280).

8. Adapter according to Claim 7 wherein the media separating diaphragm (4) comprises a flexible valve flap (40) which closes said milk outlet opening (284).

9. Adapter according to one of Claims 1 to 8, wherein the media separating diaphragm (4) comprises a circular circumferential edge (45) which is realized in a thicker manner than the flattened spherical calotte of the media separating diaphragm (4) and which is provided at least in portions with a positioning ring (43).

10. Adapter according to one of Claims 1 to 9, wherein the adapter (2) comprises a basic body (28) on the breast shield side and a cover (24) remote from the breast shield, wherein the chamber (280) is arranged in the basic body (28) and wherein the cover (24) closes the opening (283) of the chamber (280) remote from the breast shield.

11. Adapter according to Claims 9 and 10, wherein the basic body (28) comprises a circular circumferential first sealing edge (281), over which the media separating diaphragm (4) can be pulled, wherein the at least partially circumferential positioning ring (43) surrounds said first sealing edge (281).

12. Adapter according to one of Claims 9 to 11, wherein the cover (24) comprises a circular circumferential second sealing edge (29), over which the media separating diaphragm (4) can be pulled by way of its circumferential edge (45), wherein the circumferential edge (45) abuts in a sealing manner against the second sealing edge (29).

13. Adapter according to Claims 11 and 12, wherein, with the cover (24) closed, the positioning ring (43) of the media separating diaphragm (4) forms an outside surface of the adapter (2) between the cover (24) and the basic body (28).

14. Adapter according to one of Claims 10 to 13, wherein the cover (24) is arranged on the basic body (28) so as to be pivotable about one single pivot axis (240).

## Revendications

1. Adaptateur (2) pour une téterelle (1) d'un tire-lait en vue du pompage de lait maternel, la téterelle (1) pouvant être retenue dans l'adaptateur (2) ou étant connectée d'une seule pièce avec celui-ci,
l'adaptateur (2) présentant une connexion de succion (23) pour la connexion à une pompe à vide (6) et une connexion de lait (22) pour la connexion à un réservoir de collecte de lait (7), l'adaptateur (2) présentant une chambre (280) de laquelle sortent la connexion de succion (23) et une ouverture de sortie de lait (284),
la chambre (280) présentant en outre une ouverture (282) du côté de la téterelle, conduisant à la téterelle (1), ayant un premier diamètre et une ouverture (283) éloignée de la téterelle, ayant un deuxième diamètre,
et l'adaptateur (2) présentant en outre une membrane flexible de séparation de milieux (4), qui est reçue dans la chambre (280) et qui sépare la chambre (280) en une région côté pompe et une région côté téterelle afin de transférer une dépression générée par la pompe à vide (6) dans la téterelle (1) et afin de protéger la pompe à vide contre la contamination, la chambre (280) se rétrécissant vers l'ouverture (282) côté téterelle, la chambre (280) présentant la forme d'une calotte sphérique qui est coupée dans sa région se rétrécissant, la membrane de séparation de milieux (4) présentant essentiellement la forme d'une calotte sphérique aplatie avec un côté supérieur plat (411) et le côté supérieur plat (411) de la membrane de séparation de milieux (4) étant tourné vers l'ouverture côté téterelle (282) de la chambre (280), **caractérisé en ce que** la membrane de séparation de milieux (4) présente un renfoncement (42) qui forme une partie d'un canal de lait (14) entre l'ouverture (282) côté téterelle et le la connexion de lait (22).

2. Adaptateur selon la revendication 1, dans lequel le côté supérieur plat (411) de la membrane de séparation de milieux (4) présente un diamètre qui correspond approximativement au diamètre de l'ouverture (282) côté téterelle.

3. Adaptateur selon l'une quelconque des revendications 1 et 2, dans lequel la chambre (280) se rétrécit en continu dans la région de l'ouverture (282) côté téterelle jusqu'à l'ouverture.

4. Adaptateur selon l'une quelconque des revendications 1 à 3, dans lequel la membrane de séparation de milieux (4) s'applique sans sollicitation essentiellement contre un côté intérieur de la chambre (280), sans sollicitation par une dépression.

5. Adaptateur selon l'une quelconque des revendications 1 à 4, dans lequel la membrane de séparation de milieux (4) est réalisée sous forme flexible.

6. Adaptateur selon l'une quelconque des revendications 1 à 5, dans lequel le renfoncement (42) se trouve dans une région de transition entre le côté supérieur plat (411) et une surface d'enveloppe courbe de la calotte sphérique aplatie de la membrane de séparation de milieux (4).

7. Adaptateur selon l'une quelconque des revendications 1 à 6, dans lequel l'ouverture de sortie de lait (284) est disposée dans la région en forme de calotte sphérique de la chambre (280).

8. Adaptateur selon la revendication 7, dans lequel la membrane de séparation de milieux (4) présente un clapet de valve flexible (40) qui ferme cette ouverture de sortie de lait (284).

9. Adaptateur selon l'une quelconque des revendications 1 à 8, dans lequel la membrane de séparation de milieux (4) présente un bord périphérique circulaire (45) qui est plus épais que la calotte sphérique aplatie de la membrane de séparation de milieux (4) et qui est pourvu au moins en partie d'un anneau de positionnement (43) .

10. Adaptateur selon l'une quelconque des revendications 1 à 9, l'adaptateur (2) présentant un corps de base (28) du côté de la téterelle et un couvercle (24) éloigné de la téterelle, la chambre (280) étant disposée dans le corps de base (28) et le couvercle (24) fermant l'ouverture (283) de la chambre (280) éloignée de la téterelle.

11. Adaptateur selon les revendications 9 et 10, dans lequel le corps de base (28) présente un premier bord d'étanchéité périphérique circulaire (281) par-dessus lequel la membrane de séparation de milieux (4) peut être repliée, l'anneau de positionnement au moins partiellement périphérique (43) entourant ce premier bord d'étanchéité (281).

12. Adaptateur selon l'une quelconque des revendications 9 à 11, dans lequel le couvercle (24) présente un deuxième bord d'étanchéité périphérique circulaire (29) par-dessus lequel la membrane de séparation de milieux (4) peut être repliée avec son bord périphérique (45), le bord périphérique (45) s'appliquant de manière hermétique contre le deuxième bord d'étanchéité (29) .

13. Adaptateur selon les revendications 11 et 12, dans lequel l'anneau de positionnement (43) de la membrane de séparation de milieux (4), lorsque le couvercle (24) est fermé, forme une surface extérieure de l'adaptateur (2) entre le couvercle (24) et le corps de base (28).

14. Adaptateur selon l'une quelconque des revendications 10 à 13, dans lequel le couvercle (24) est disposé sur le corps de base (28) de manière à pouvoir pivoter autour d'un axe de pivotement unique (240).
